Europäisches Patentamt

European Patent Office

Office européen des brevets

(11) Publication number : **0 501 763 A1**

# EUROPEAN PATENT APPLICATION

(21) Application number : 92301593.7

(51) Int. Cl.⁵ : **A61K 31/40, A61K 47/48**

(22) Date of filing : 26.02.92

(30) Priority : 26.02.91 GB 9104042

(43) Date of publication of application :
02.09.92 Bulletin 92/36

(84) Designated Contracting States :
AT BE CH DE DK ES FR GB GR IT LI LU NL PT SE

(71) Applicant : **ABBOTT LABORATORIES LIMITED**
**Queenborough Kent, ME11 5EL (GB)**

(72) Inventor : **Honeysett, Robert Alan**
**Joskins, Pilgrims Way, Old Wives Lees**
**Canterbury, Kent (GB)**
Inventor : **Feely, Liam Christopher**
**20 Lingfield Road, Borough Green**
**Near Sevenoaks, Kent (GB)**
Inventor : **Hoadley, Thomas Herbert**
**Windrush, 36 Darlington Drive, Minster**
**Isle of Sheppey, Kent (GB)**
Inventor : **Sims, Edward Eric**
**97 Lombardy Drive**
**Maidstone, Kent ME14 5TB (GB)**

(74) Representative : **Hayward, Denis Edward Peter**
**Lloyd Wise, Tregear & Co. Norman House**
**105-109 Strand**
**London WC2R 0AE (GB)**

(54) **Taste-masked buflomedil preparation.**

(57)   A taste-masked preparation of the drug buflomedil is provided in the form of a cation-exchange resin with the drug bonded thereto. This buflomedil resinate is prepared by soaking the resin in a solution of the free drug, separating and washing the resinate to remove any unbonded free drug and then drying. To make a dosage form for oral administration the dried resinate is deaggregated by milling and then blended with suspending and bulking agents and known excipients, such as one or more sweeteners and a glidant.

EP 0 501 763 A1

This invention is concerned with preparations of the drug buflomedil for oral administration.

The pharmaceutical compound buflomedil, administered orally as the hydrochloride salt, is very useful as a vasodilator, especially in the treatment of geriatric patients. It is, however, included amongst those drugs that, because of rapid elimination or metabolism, need to be administered frequently, i.e. according to a daily regimen, and it has the disadvantage of possessing an extremely bitter taste.

It is therefore an object of this invention to achieve a formulation of the drug for oral administration that is more acceptably palatable.

According to the invention, a taste-masked buflomedil preparation is obtained by employing an ion-exchange resin to convert the buflomedil hydrochloride into a buflomedil resinate complex. This can then be blended with sweeteners and/or other pharmaceutical excipients to produce the final dosage form of the drug which may, for instance, be constituted as a powder contained in a sachet.

The invention thus provides a taste-masked and palatable presentation of buflomedil that comprises the drug bound to a cation-exchange resin. Buflomedil hydrochloride can be bound to cation-exchange resins by soaking the resin in an aqueous solution of the drug; this is followed by washing and drying. Levels of loading of the buflomedil on to the resin up to 60% w/w have been obtained and the resultant buflomedil resin complex is virtually tasteless. Amongst suitable resins are Amberlite IRP 69 (a pharmaceutical grade of sodium polystyrene sulphonate) and Amberlite IRP 64 (a carboxylate form of the styrene polymer), these being obtainable from Rohm and Haas Co. of Philadelphia, U.S.A. Other suitable anionic resins are also available from other sources.

After oral administration, the drug is liberated _in vivo_ as a consequence of small cations in the gastro-intestinal tract ($H^+$, $Na^+$ etc.) competing for the resin binding sites and displacing the buflomedil. Bioavailability studies conducted originally with dogs and latterly with human volunteers have shown that the buflomedil is substantially as bioavailable from the resinate complex as from the hydrochloride in solution.

Examples of specific methods of preparation of the buflomedil resinate complex and subsequent formulation of the dosage units will now be described in some detail by way of illustration only.

Preparation of Buflomedil Resinate

Example 1 (To produce up to 5kg of resinate)

A quantity of buflomedil hydrochloride is dissolved in purified water at 10% w/v - this concentration is not critical. Amberlite IRP 69 ion-exchange resin is then suspended in the buflomedil solution at 80g per litre of solution and the suspension stirred for 1 hour and allowed to settle for 1 hour. The supernatant liquid is decanted and the residue is then suspended in a fresh batch of the buflomedil solution and the stirring, settling and decanting repeated as before. This repetition achieves a higher level of ion-exchange than one pass. The times and ratios are not critical but overall ion-exchange efficiency is dependent on time, concentration and temperature and the presence of competing ions, in this case $Na^+$.

After the ion-exchange treatment, the resinate is rinsed with sufficient purified water to remove unexchanged buflomedil hydrochloride. Proper rinsing to remove the free drug is essential. The end point is typically when the eluent no longer has a bitter taste and free drug is not identified analytically. The resinate is separated by filtering and dried to a cake in a vacuum oven at 50°C. The drying can be carried out by other suitable methods, such as forced hot air flow, elevated temperature fluidisation, etc. Finally, the dried material is deaggregated using a suitable mill, typically a hammer mill, and screened through a 0.8mm aperture screen. This requires care since, as with many milling and screening processes, there is some explosion risk.

Except for the drying step, all the procedures are carried at ambient temperatures in the range 16°C - 24°C. Assays show that the product obtained by this method of preparation consistently has a concentration of the equivalent of 42 - 52 grams of buflomedil hydrochloride per 100g of buflomedil resinate.

Example 2 (To produce a 20kg batch of resinate)

A solution of buflomedil hydrochloride is prepared as in Example 1. A suspension of the ion-exchange resin Amberlite IRP 69, in either a portion of the buflomedil solution or in purified water, is then pumped into a centrifuge and processed in contact with the buflomedil solution which is recirculated through the centrifuge to effect the ion-exchange. Times and flow rates are not critical to the exchange process but typically 3 to 6 hours processing is carried out at a flow rate of 700-900 litres/hr.

The resinate is then washed to remove free buflomedil by spraying a sufficient quantity of purified water through the centrifuge. After spin-drying, the resinate is unloaded from the centrifuge into a vacuum oven and dried at 50°C under maximum vacuum. As in Example 1, alternative drying methods can be used.

All procedures, except the drying step, are carried out at ambient temperatures in the same range as before. The resinate product of Example 2 assays at the equivalent of 38-42 grams of buflomedil hydrochloride per 100g. of buflomedil resinate.

In both the above two Examples the ion-exchange resin employed was Amberlite IRP 69. This is preferred to Amberlite IRP 64 which requires pre-conditioning from the ($H^+$) to the ($Na^+$) form prior to the ion exchange. Experimentally, this resin gave lower buflomedil loadings on the resinate complex of 20-30% w/w.

Dose Formulation

Buflomedil hydrochloride is usually prescribed at a does of 150-600mg daily. The buflomedil resinate complexes according to the present invention are readily capable of providing dosages anywhere in a range up to 1000mg buflomedil hydrochloride equivalent per dose.

A typical dose formulation is as follows:-

| | |
|---|---|
| Buflomedil resinate | 0.600g |
| Modified Starch (suspending agent) | 1.000g |
| Sorbitol (Bulking agent and sweetener) | 3.140g |
| Colloidal Silicon Dioxide (Glidant) | 0.035g |
| Aspartame (High intensity sweetener | 0.025g |
| Flavour | 0.200g |
| Total | 5.000g |

This is equivalent to 300mg buflomedil hydrochloride assuming a 50% w/w loading of the drug on the resin. The sorbitol level can be adjusted to suit changes in the effective strength of the buflomedil resinate.

To prepare the formulation, the ingredients may first be screened, if necessary, to ensure deaggregation. The flavour, glidant and high intensity sweetener are then preblended dry with a portion of the bulking agent. This is to ensure uniformity of blending of the small quantities involved and may be carried out manually in a polythene bag. Next the preblend, the starch and the balance of the bulking agent are machine blended dry until uniformly homogeneous. Any suitable dry blender may be used - planetary mixers have been demonstrated to achieve uniform blending after a blending time of 10-25 minutes. The blended mixture is then filled into suitable containers, e.g. sachets, in 5g. quantities to give drug doses of 300mg. per sachet, for which purpose any suitable powder dose dispenser can be used.

To achieve an acceptable mouthfeel and avoid the dose seeming to be gritty when swallowed, it is important that the particle size and the excipients be selected to reduce this grittiness to a minimum.

The practice of the invention is of course, not limited to use of the excipients listed above since a wide range are available. Amongst these are the following: as suspending agents, other starches, celluloses, xanthan gum, Carrageenan gum, carbomer; as bulking agents, sucrose, isomalt, mannitol, xylitol; as glidants, talc, precipitated silicon dioxide; as sweetners, such pharmaceutically acceptable sweetening compounds as are available in commerce. As a general constraint, however, small cations are undesirable. Suggested ranges for the suspending agent and the bulking agent in the 5g formulation are 0.1g to 2.0g and 1g to 4g, respectively.

Other dosage forms for oral administration that can utilise buflomedil resinate as the active ingredient include liquid suspensions and chewable tablets. Common excipients used in such products include glucose, lycasin, lactose and dextrose. In fact, any conventional pharmaceutical excipient can be employed so long as it is not a water soluble compound having a small cation such as $Na^+$, $H^+$, $Ca^{++}$ etc., which latter might result in premature displacement of the buflomedil on the ion exchange resin.

Tests were conducted to examine the effects of the resin particle size, buflomedil solution concentration reaction temperature and reaction time on the loading of the buflomedil drug on the resin, as follows:-

Effect of resin particle size

| Resinate size fraction | % Buflomedil loading on to resin | |
| --- | --- | --- |
| | Lot 31-017VC | Lot 38-013VC |
| Less than 50 um | 54.8 | 56.1 |
| 50 um – 80 um | 57.8 | 55.4 |
| 80 um – 125 um | 52.3 | 50.5 |

According to this study, the drug loading is not significantly affected by the resin particle size over the range studied.

Effect of Buflomedil Solution Concentration (laboratory scale)

| Concentration of Buflomedil HCl solution, % w/v | % Buflomedil loading onto resin |
| --- | --- |
| 10 | 33.8 |
| 15 | 43.1 |
| 20 | 46.9 |
| 30 | 48.6 |

According to this, the drug loading increases as the solution concentration increases but the change is minimal above a solution concentration of 20% w/v.

Effect of reaction temperature (laboratory scale)

| Temperature | % Buflomedil loading on to resin |
| --- | --- |
| 25°C | 41.3 |
| 50°C | 41.0 |
| 70°C | 40.4 |

Thus, there is no significant effect of reaction temperature on loading over the range studied.

Effect of reaction time (laboratory scale)

| Time | % Buflomedil loading on to resin |
| --- | --- |
| 1 hour | 41.3 |
| 2 hours | 43.7 |
| 3 hours | 42.2 |

Thus, the loading of buflomedil on to the ion-exchange resin occurs rather rapidly and extending the reaction time does not achieve any significant increase in loading.

The invention is not to be considered as in any way limited to the procedures set out in the specific examples hereinbefore described which are illustrative only. Thus, other ion exchange procedures, such as column exchange, for example, may be employed.

**Claims**

1. Process for making a taste-masked buflomedil preparation comprising the step of employing an ion-exchange resin to convert buflomedil hydrochloride into a buflomedil resinate complex.

2. Process according to Claim 1, wherein the buflomedil resinate complex is blended with sweeteners and/or other optional pharmaceutical excipients to produce a dosage form of the drug.

3. Process according to Claim 1 or Claim 2, wherein the buflomedil hydrochloride is bound to a cation-exchange resin by soaking the resin in an aqueous solution of the drug, this step being followed by washing to remove any remaining free drug and drying.

4. Process according to Claim 3, wherein the resin employed is a pharmaceutical grade of sodium polystyrene sulphonate or a carboxylate form thereof.

5. Process according to Claim 3 or Claim 4, wherein the resin is soaked successively in two or more fresh batches of the buflomedil solution before drying.

6. Process according to Claim 3 or Claim 4, wherein the resinate is introduced in suspension into a centrifuge and the buflomedil solution is circulated through the centrifuge to effect the ion exchange after which wash water is passed through the centrifuge and then the washed resinate is spin dried.

7. Process according to any of Claims 3 to 6, wherein the drying of the resinate is completed in a vacuum oven after which the resinate is deaggregated by milling and screening.

8. Process according to any of Claims 3 to 7, wherein the resin is soaked in a 10 to 30%, and preferably about 20%, w/v solution of the buflomedil hydrochloride for at least about 1 hour and, except for the oven-drying, the process steps are performed at ambient temperature.

9. A taste-masked buflomedil resinate preparation comprising the drug buflomedil bound to a cation-exchange resin.

10. A dosage form of buflomedil comprising the buflomedil resinate preparation of Claim 9, dry-blended to a uniformly homogeneous state with a pharmaceutically-acceptable suspending agent, bulking agent, one or more sweeteners and optionally a glidant, except that water-soluble compounds having a small cation, such as $Na^+$, $H^+$, $Ca^{++}$, are avoided the said blended materials being reduced to a size at which there is no gritty feeling in the mouth when a dose is administered orally.

European Patent Office

**EUROPEAN SEARCH REPORT**

Application Number

EP 92 30 1593

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | STN FILE SERVER & FILE MEDLINE, accession no. 86187320; D. PICCINELLI et al.: "[Acute toxicity of (2,4,6-trimethoxy) phenyl-3 (pyrrolidinyl-1) propyl-ketone chlorhydrate ( ***buflomedil*** ) and of metacrylic copolymers (eudragit) both alone and in association in the mouse and rat]", & BOLL. SOC. ITAL. BIOL. SPER, (1985 DEC. 30) 61 (11-12) 1579-85 * Whole abstract * | 1-10 | A 61 K 31/40<br>A 61 K 47/48 |
| Y | EP-A-0 431 759 (GLAXO GROUP) * Page 2, lines 13-32; claims * | 1-10 | |
| A | STN FILE SERVER & FILE CHEMICAL ABSTRACTS, vol. 115, no. 4, abstract no. 35625c, Columbus, Ohio, US; C.G. WILSON et al.: "Predictive modeling of the behavior of a controlled release buflomedil hydrochloride formulation using scintigraphic and pharmacokinetic data", & INT. J. PHARM., 72(1), 79-86 * Whole abstract * | 1 | TECHNICAL FIELDS SEARCHED (Int. Cl.5)<br><br>A 61 K |

The present search report has been drawn up for all claims

| Place of search | Date of completion of the search | Examiner |
|---|---|---|
| THE HAGUE | 10-04-1992 | BERTE M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)